(19) Europäisches Patentamt
European Patent Office
Office européen des brevets

(11) Publication number: 0 306 236 B1

(12) # EUROPEAN PATENT SPECIFICATION

(45) Date of publication of patent specification: **28.04.93**

(51) Int. Cl.[5]: **A61K 9/22**, A61K 7/00, A61K 7/46, A61K 9/50

(21) Application number: **88307947.7**

(22) Date of filing: **26.08.88**

(54) **Controlled release formulations.**

(30) Priority: **31.08.87 US 91641**

(43) Date of publication of application:
**08.03.89 Bulletin 89/10**

(45) Publication of the grant of the patent:
**28.04.93 Bulletin 93/17**

(84) Designated Contracting States:
**CH DE FR GB LI**

(56) References cited:
**EP-A- 0 143 608**
**FR-A- 2 352 547**
**US-A- 4 690 825**

(73) Proprietor: **Advanced Polymer Systems, Inc.**
**3696C Haven Avenue**
**Redwood City, CA 94063(US)**

(72) Inventor: **Katz, Martin,**
**5 Whitney Court**
**Menlo Park, CA 94025(US)**
Inventor: **Cheng, Chung-Heng,**
**1162 Robalo Court,**
**San Jose, CA 95131(US)**
Inventor: **Won, Richard,**
**3787 Nathan Way**
**Palo Alto, CA 94303(US)**
Inventor: **Leong, Helen C.,**
**146 Atherton Avenue,**
**Atherton, CA 94025(US)**

(74) Representative: **Stuart, Ian Alexander et al**
**MEWBURN ELLIS & CO. 2/3 Cursitor Street**
**London EC4A 1BO (GB)**

EP 0 306 236 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to controlled release compositions comprised of porous polymeric microbead carriers retaining within their pores various types of impregnants. For example, the impregnants may comprise one or more of: topical formulations useful in the promotion of hair growth; topical compositions used in the treatment of acne; vitamins and vitamin derivatives (particularly retinoids); and epidermal lipid replacement substances. The invention further relates to methods of preparing such compositions and their methods of use.

EP-A-0 143 608 discloses a method for making beads that comprise a polymeric matrix that provides slow release of a lipophilic compound; the matrix is a hydrophilic monomer, selected from acrylic acid and methacrylic acid. The method comprises bead polymerisation of a homogeneous blend of the compound in polymerisable monomer or swelling the polymer matrix by a swelling agent for the matrix and carrying the compound into the swollen matrix.

### 1.1 Topical Formulations for the Promotion of Hair Growth

Minoxidil has recently been reported to exhibit beneficial properties for the promotion of hair growth. See, e.g., U.S. Patent No. 4,139,619, the disclosure of which is specifically incorporated herein by reference. Minoxidil is a piperidinopyrimidine and is described in the Merck Index (10th Edition) published by Merck & Co., Inc., Rahway, New Jersey at pages 888-889, the disclosure of which is specifically incorporated herein by reference. While Minoxidil is packaged in a tablet or capsule for use in the treatment of hypertension, a delivery vehicle for the topical application to a skin surface is needed. In order to maximize the benefit of such application, the vehicle should allow absorption through the skin surface to which it is applied. Additionally, it would be preferable to provide a time release mechanism for absorption of the active ingredient.

### 1.2 Topical Compositions used in the Treatment of Acne

Acne is a pleomorphic skin disease characterized by blackheads, whiteheads, papules, pustules, cysts, and various sized nodules and scars which, in the inflammatory stage of the disease, are contaminated with bacteria such as Propionibacterium acnes. The disease involves the pilosebaceous units of the dermis which consist of the sebaceous follicle which include the sebaceous glands and ducts and small hairs.

Sebum (skin oil) is a complex mixture of fats and waxes liberated by the breakdown of the sebaceous cells. The production of sebum is intimately associated with the pathology of acne. Obstruction of the opening of the sebaceous follicle by a comedone, a solid horny mass or plug made up of keratinized cells and commonly referred to as whiteheads or blackheads, may block or stagnate sebum flow through the sebaceous follicle. This blockage leads secondarily to rupture of the follicular contents (sebum) into the dermis, and then to perfolliculitis. This provokes an inflammatory response which leads to the formation of pustules (pimples) when the rupture is small, and cystic nodules with complete rupture. A scar may eventually form, dependent on the depth and extent of the inflammatory response.

Benzoyl peroxide is an oxidizing agent believed to be one of the most effective topical nonprescription medications available for acne. Benzoyl peroxide is one of several topical agents used in treating acne to cause irritation and desquamation, thereby preventing closure of the pilosebaceous orifice. Salicylic acid is a known keratolytic and antibacterial agent also used in the treatment of acne.

Benzoyl peroxide is soluble in nearly all organic solvents, slightly soluble in alcohols and slightly soluble in water. It has a melting point of 103 °C to 105 °C and a specific gravity of 1.3340 at 25 °C. Salicylic acid has a melting point of 158-161 °C and a specific gravity of 1.443 (20/4 °C). Salicylic acid is soluble in acetone, alcohol, ether and benzene, and slightly soluble in water.

The irritant effect of these peeling agents causes an increased turnover rate of epithelial cells lining the follicular duct, which increases sloughing. The desired effect is to reduce the tendency of the skin to form new comedones and to loosen the structure of the formed comedones and aid in their extrusion.

In the past, benzoyl peroxide has been administered to the skin in the form of gels, creams, lotions or ointments in concentrations of 2.5%-5.0% and 10%. There are several disadvantages to prior methods of delivering benzoyl peroxide to affected areas. Strong concentrations of benzoyl peroxide cannot be used because high concentrations of the topical agent can cause localized reactions such as stinging and burning. Benzoyl peroxide is also a strong bleaching agent, and can permanently discolor sheets, pillowcases, towels, clothing or any other colored textiles if a liquid or gel containing the agent is spilled or otherwise contacts such articles.

Problems have been encountered in attempting to combine salicylic acid with benzoyl peroxide. These problems are detailed in U.S. Patent No. 4,514,385, issued April 30, 1985 to Damani et al. Damani et al. suggests dispersing or suspending finely divided particles of benzoyl peroxide and salicylic acid in a particular class of aqueous carboxy vinyl polymer (carboxypolymethylene) gels in the absence of alkaline neutralizing agents which would tend to react with salicylic acid. The resulting products, characterized as creamy gels, are applied topically or in toiletries such as shampoos, soap bars and detergent bars used in washing the skin.

### 1.3 Vitamins and Vitamin Derivatives

Vitamins are chemically unrelated, fairly complex organic substances that are essential in small amounts for the maintenance of normal metabolic functions. Vitamins are not synthesized within the human body and must therefore be furnished from exogenous sources. Natural sources of vitamins include plant and animal tissue.

Vitamins can be broadly categorized as either fat-soluble or water-soluble. Vitamins A, D, E and K are fat soluble and are absorbed in association with lipids. Vitamins $B_1$, $B_2$, $B_5$, $B_{12}$, $B_{15}$, $B_{17}$, C, niacin, folic acid, pantothenic acid, biotin, bioflavonoids, choline, inositol and F are examples of water-soluble vitamins.

Vitamins A, D and E, and derivatives thereof, including esters such as vitamin A palmitate, vitamin E acetate and vitamin E palmitate and substances which contain such vitamins and vitamin derivatives, such as fish oils, have long been known to be useful for various topical therapeutic and cosmetic purposes. Creams, ointments and powders containing vitamins A and D as such or in cod liver oil have been used for the prevention and treatment of prickly heat and diaper rash in infants. Vitamin E and vitamin E esters, such as the acetate and palmitate, have been used, typically in creams, ointments, lotions, oils and other cosmetic formulations, as skin moisturizers and smoothers, to treat chronic skin diseases, to aid in healing burns and wounds, to reduce scarring from wounds, as topical anti-inflammatory agents, and to protect the skin from damage from ultraviolet rays.

Vitamin-containing therapeutic and cosmetic creams, ointments, lotions, oils and like formulations feel greasy or oily when applied, and often leave residues which are difficult to remove from the skin. Vitamin-containing powder preparations enable only limited amounts of vitamin to penetrate the skin.

Vitamins are also used as additives in preparations primarily intended for purely cosmetic use, such as lip balms, lipsticks, blushers, eyeshadows and foundations.

Retinoids are a group of compounds consisting of vitamin A and its analogs. In addition to their effectiveness in enhancing vision and fertility, the compounds in this group are effective as keratolytic agents, and as such are used topically to retard and ameliorate photoaging of facial skin and senile keratosis, to treat acne vulgaris, and to treat warts.

Vitamin A itself is a term used to designate several biologically active compounds, principally the alcohols retinol, whose chemical name is 3,7-dimethyl-9-(2,6,6-trimethyl-1-cyclohexen-1-yl)-2,4,6,8-nonatetraenol, and 3-dehydroretinol. Other retinoids include the corresponding acids, aldehydes, esters and aromatic derivatives of these. The most common of the acids is retinoic acid, or 3,7-dimethyl-9-(2,6,6-trimethyl-1-cyclohexen-1-yl)-2,4,6,8-nonatetraenoic acid, which has the structural formula:

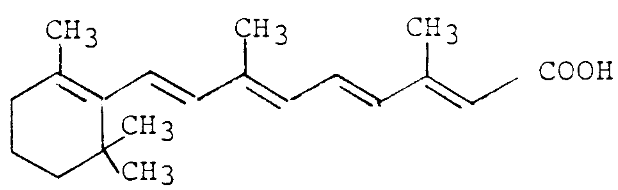

This formula shows the all-trans-form, which is also known by the common name tretinoin. Other acids within the class include the 9,10-cis-form and the 13-cis-form. Examples of esters are the methyl ester and the ethyl ester. The acids are crystalline under ambient conditions, soluble in fats and alcohols. The esters are liquids under ambient conditions. The acids and their derivatives are of particular interest for their high therapeutic value.

For topical application, retinoids are typically formulated as creams, ointments, oils and the like. In such formulations, however, retinoids rapidly degrade and lose activity. In addition, moderate to severe skin irritation frequently results from the use of these formulations. Still further, these formulations usually feel

oily or greasy when applied, and tend to leave residues on the skin which are difficult to remove.

**1.4 Epidermal Lipid Replacement Substances**

Squalane and squalene are oils used as replacements for the natural epidermal lipids of the skin, which become depleted due to aging and general conditions of skin dryness. These oils are used for pharmaceutical and cosmetic purposes, and are applied directly to the skin, reconstituting the outer layers of the skin. They thus find utility as skin emollients, skin lubricants and sebum replacement agents. They are also used as fragrance fixatives and carriers for lipid soluble drugs. Examples of such drugs are vitamins such as A, D, and E, and keratolytic agents such as retinoic acid. In addition to these properties, squalane and squalene are further characterized by low freezing points, extending their utility to situations where other candidates cannot be used.

Squalene is a naturally occurring substance found in large quantities in shark liver oil and in smaller amounts (0.1-0.7%) in olive oil, wheat germ oil, rice bran oil and yeast. Its chemical name is 2,6,10,15,19,23-hexamethyl-2,6,10,14,18,22-tetracosahexaene, and it has the structural formula:

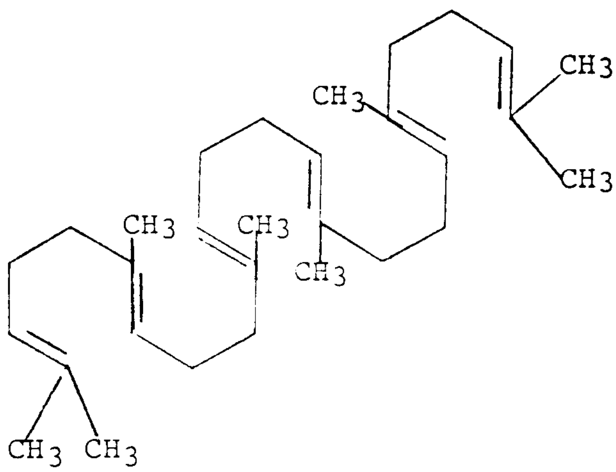

Squalane is fully hydrogenated squalene, or 2,6,10,15,19,23-hexamethyltetracosane, with the structural formula:

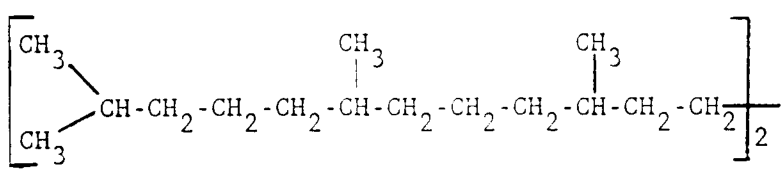

There are certain disadvantages to the use of these compounds. Squalene absorbs oxygen, and upon doing so becomes highly viscous. It is also prone to oxidation and the resulting development of a rancid odor. Squalane is somewhat more stable but not completely. Neither are water-soluble and hence dispersible in an aqueous system, and both have an inherently oily character, leaving a residue on the skin with an unpleasant feel.

**SUMMARY OF THE INVENTION**

According to the invention in one aspect there is provided a topical composition comprising solid particles containing a substantially continuous non-collapsible network of pores open to the exterior of said particles, and an impregnant retained inside said pores, wherein said impregnant is optionally in a solvent and comprises one or more of: benzoyl peroxide; retinol, 3-dihydroretinol, retinoic acids, retinoic aldehydes, retinoic acid esters, and aromatic derivatives thereof; minoxidil; squalane; and squalene; prepared by:

(a) forming said particles by suspension polymerisation in the presence of a porogen so that porous particles with the porogen in the pores are formed;

(b) extracting the porogen from the pores; and

(c) impregnating the pores with said impregnant, and wherein said solid particles are substantially spherical in shape, are formed from a cross-linked polymer, having an average diameter of about 10 µm to about 40 µm, having a total pore volume of about 0.1 ml/g to about 2.0 ml/g, having a surface area of about 20 $m^2/g$ to about 200 $m^2/g$, and having an average pore diameter of about 0.003 to about 1.0 µm.

## 2.1 Topical Formulations for the Promotion of Hair Growth

A novel formulation for topically delivering active ingredients useful for promoting hair growth has now been developed with significant advantages over preexisting formulations. In accordance with the present invention, porous particles of an inert solid material contain the active ingredient, alone or in solution, retained inside the pores of the particles by capillary forces. The pores are interconnected and open to the particle surface, permitting full diffusion outward of the retained active ingredients. The particles, which are preferably and most conveniently in the form of microspheres, are used either alone as a powder or as a dispersion in a suitable vehicle in a form resembling those of conventional skin preparations such as liniments, gels, lotions or ointments.

## 2.2 Topical Compositions used in the Treatment of Acne

A novel formulation for topically delivering active ingredients used in the treatment of acne has now been developed with significant advantages over preexisting formulations. In accordance with the present invention, porous particles of an inert solid material contain benzoyl peroxide and optionally salicylic acid, alone or in solution, retained inside the pores of the particles by capillary forces. The pores are interconnected and open to the particle surface, permitting full diffusion outward of the retained active ingredients. The particles, which are preferably and most conveniently in the form of microspheres, are used either alone as a powder or as a dispersion in a suitable vehicle in a form resembling those of conventional skin preparations such as liniments, gels, lotions or ointments.

## 2.3 Vitamins and Vitamin Derivatives

A novel formulation for topically delivering retinoid compositions has now been developed with significant advantages over preexisting formulations. In accordance with the present invention, porous particles of an inert solid material contain vitamins, vitamin derivatives and vitamin-containing substances comprising retinoids, alone or in solution, retained inside the pores of the particles by capillary forces. The pores are interconnected and open to the particle surface, permitting full diffusion outward of the retained active ingredients. The particles, which are preferably and most conveniently in the form of microspheres, are used either alone as a powder or as a dispersion in a suitable vehicle in a form resembling those of conventional skin preparations such as liniments, gels, lotions or ointments.

More specifically, the delivery systems of this invention constitute pre-formed, discrete, minute, high-capacity, hydrophobic, macroporous or macroreticular crosslinked, monionogenic polymer beads into whose macropores there have been incorporated vitamins and/or derivatives thereof and/or vitamin-containing substances such as cod liver oil, and the like, comprising retinoids, as such or dissolved in a suitable organic solvent, alone or together with other formulating materials. Porous particles of an inert solid material contain one or more retinoids, alone or in solution, retained inside the pores of the particles by the pore structure if solid and by capillary forces if liquid. The pores are interconnected and open to the particle surface, permitting full diffusion outward of the retained retinoids. The particles, which are preferably and most conveniently in the form of microspheres, are used either alone as a powder or as a dispersion in a suitable vehicle in a form resembling those of conventional skin preparations such as liniments, gels, lotions or ointments.

## 2.4 Epidermal Lipid Replacement Substances

A novel formulation for squalane and squalene has now been developed which overcomes the disadvantages of known formulations. In accordance with the present invention, porous particles of an inert solid material contain one or both of the oils, alone or in solution, retained inside the pores of the particles by capillary forces. The pores are interconnected and open to the particle surface, permitting full diffusion outward of the retained oils. The particles, which are preferably and most conveniently in the form of microspheres, are used either alone as a powder or as a dispersion in a suitable vehicle in a form

resembling those of conventional skin preparations such as liniments, gels, lotions or ointments.

In general, the active ingredients of various types described above diffuse out of the pores into either the vehicle if one is used or the natural bodily secretions present on one's skin at the applied area, in accordance with known principles of the diffusion of one liquid through another.

The particles function as controlled delivery systems for the active ingredients, providing a wide range of advantages over the conventional formulations. Release of the active ingredients from the pores of the beads occurs in sustained manner, providing a continuous fresh supply of active substance to the epidermal area to which the preparation has been applied. Until it is released, the active ingredient is essentially unexposed to the atmosphere, and hence contact with oxygen and the risks of oxidation and decomposition are minimal. The formulation remains stable and active for a longer period of time, enhancing its shelf life. In addition, the particles have a dry, smooth, comfortable feel to the skin.

The activity-time curve of the active ingredient is thus extended and flattened out. The magnitude of the release rate is controlled by the pore volume distribution in the microsphere itself, notably the total pore volume and the average pore diameter. Selection of the values of these parameters according to predetermined standards provides control of the release rate to desired levels. This controlled release rate enhances the continuing treatment of the epidermal area, as sebum is continually produced by the skin. This also reduces the number of times that the skin formulation must be reapplied to the affected area.

The preparations remain active for a longer period of time after having been applied to the skin than conventional formulations, due to the sustained release character. The rate of release can be accelerated at any time thereafter by manual friction to stimulate the outward diffusion of the active ingredient. Thus, deeply retained materials may be brought to the surface and made available for their activity at will, many hours after the application of the formulation, without the need for a repeat application.

A further advantage is the ability of the formulation to withstand a higher concentration of active ingredient both inside the pores themselves and in the total preparation without the magnitude of side effects previously experienced at these levels.

As will be seen from the description which follows, the present invention is applicable to a wide range of active ingredients, and the degree of control which the system imparts to the various properties of the formulation and its use provide improved formulations of the active ingredients with a wide range of utility with enhanced safety and effectiveness.

## DETAILED DESCRIPTION OF THE SPECIFIC EMBODIMENTS

The beads or microspheres used in connection with the present invention are rigid, open-pore, chemically and biologically inert particles with the impregnant held inside the pores by capillary forces. The pores are interconnected and open to the particle surface to an extent that substantially full communication is provided between the internal pore space and the exterior of the particle.

In their most convenient form, the particles are generally spherical in shape, due to the use of suspension polymerization as the method of preparation. While the microspheres may vary widely in size, those falling within the range of from about 10 to about 40 µm, will provide the best results. Microspheres within this size ranges are appealing from an aesthetic point of view by imparting a smooth feel to the touch.

The pore dimensions within the spheres may also vary widely, with optimum dimensions depending on the chemical characteristics of the polymers used as well as the diffusive characteristics of the impregnant. Different systems will thus call for different optimum ranges of pore volume distribution to obtain the most desirable properties for the overall formulation. In general, however, best results are obtained with total pore volumes ranging from about 0.1 to about 2.0 ml/g; surface areas ranging from about 20 to about 200 $m^2/g$; and average pore diameters ranging from about 0.003 to about 1.0 µm. Following conventional methods of measuring and expressing pore sizes, the pore diameters are calculated from the measurement of the surface area by B.E.T. nitrogen multipoint analysis and from the measurement of the pore volumes by the mercury intrusion method. The calculation is one commonly done by those skilled in the art.

The microspheres are conveniently formed by suspension polymerization in a liquid-liquid system. In general, a solution containing monomers, a polymerization catalyst (if used), and an inert but fully miscible liquid is formed which is immiscible with water. The solution is then suspended in an aqueous solution, which generally contains additives such as surfactants and dispersants to promote the suspension. Once the suspension is established with discrete droplets of the desired size, polymerization is effected (typically by activating the reactants by either increased temperature or irradiation). Once polymerization is complete, the resulting rigid beads are recovered from the suspension. The beads at this point are solid porous structures, the polymer having formed around the inert, water-immiscible liquid, thereby forming the pore

network. The liquid has accordingly served as a porogen, or pore-forming agent, and occupies the pores of the formed beads.

Certain impregnants may serve as the porogen, in which case the porous beads recovered from the suspension immediately after polymerization are substantially ready for use, following removal of surface moisture, and any further processing steps of this nature. In these cases, microsphere formation and incorporation of the impregnant is performed in a single step. This may accordingly be termed a one-step procedure. Those impregnants which are capable of serving as porogens will be liquid impregnants meeting the following criteria:

1. They are either fully miscible with the monomer mixture or capable of being made fully miscible by the addition of a minor amount of non-water-miscible solvent;
2. They are immiscible with water, or at most only slightly soluble;
3. They are inert with respect to the monomers, and stable when in contact with any polymerization catalyst used and when subjected to any conditions needed to induce polymerization (such as temperature and radiation); and
4. They are normally liquids or have melting points below the polymerization temperature. Solids can frequently be converted to liquid form by being dissolved in a solvent or by forming eutectic mixtures.

When using this method, the steps must be performed under an inert atmosphere such as nitrogen. If a polymerization catalyst is used, it must be one which does not oxidize the impregnant, if the latter is susceptible to oxidation. Azo catalysts are examples of such catalysts. Also, polymerization temperatures are best held within a moderate range.

But the present invention concerns an alternative to the one-step procedure, wherein the impregnant may be placed inside the pores of pre-formed dry porous polymer beads. The product is thus prepared in two steps performed in sequence, the polymerization being performed first with a substitute porogen which is then removed and replaced by the desired impregnant. Materials suitable as substitute porogens will be substances which meet the same four criteria listed above for porogen impregnants.

This covers a wide range of substances. Preferred among these are hydrocarbons, particularly inert, nonpolar organic solvents. Some of the most convenient examples are alkanes, cycloalkanes, and aromatics. Examples of such solvents are alkanes of 5 to 12 carbon atoms, straight or branched chain, cycloalkanes of 5 to 8 carbon atoms, benzene, and alkyl-substituted benzenes such as toluene and the xylenes. Removal of the porogen may then be effected by solvent extraction, evaporation, or similar conventional operations.

A further advantage of the use of this two-step process is that it permits the removal of unwanted species from the polymerized structures prior to incorporation of the impregnant. Examples of unwanted species include unreacted monomers, residual catalyst, and surface active agents and/or dispersants remaining on the sphere surfaces. A further advantage of this technique is that it permits one to select the amount and type of porogen as a means of controlling the pore characteristics of the finished bead. One is thus no longer bound by the limitations of the impregnant as it affects the structure of the bead itself. This permits partial rather than full filling of the pores with the oil, and further control over pore size and distribution by selection among swelling and non-swelling porogens.

Extraction of the porogen and its replacement with (i.e., impregnation of the dry bead with) the impregnant in the two-step procedure may be effected in a variety of ways, depending on the chemical nature of the porogen and its behavior in combination with that of the other species present. The beads are first recovered from the suspension by filtration, preferably using vacuum filtration apparatus (such as a Buchner funnel). The beads are then washed with an appropriate solvent to remove organic species not bound to the polymer, including surfactants having deposited on the bead surfaces from the aqueous phase, unreacted monomers and residual catalysts, and the porogen itself. An example of such a solvent is isopropanol, either alone or in aqueous solution. Once washing is complete, the solvent itself is removed by drying, preferably in a vacuum.

In certain cases, an alternative method of extraction may be used i.e., where the porogen, unreacted monomer and water will form an azeotrope. In these cases, steam distillation is an effective way of extracting porogen from the beads. This again may be followed by drying under vacuum.

Once the beads are rendered dry and free of the substitute porogen and any unwanted organic materials, they are impregnated with the impregnant according to conventional techniques. The most convenient such technique is contact absorption. Solid active ingredients are first dissolved in a solvent, and the resulting solution is absorbed by the beads. The solvent may either be retained in the finished product or removed by conventional means such as evaporation or extraction using a further solvent. For those solid ingredients having limited solubility in a particular solvent, high contents in the finished bead can be attained by repeated absorptions each followed by solvent removal.

The polymerization process and the various parameters and process conditions involved in the polymerization can be selected and adjusted as a means of controlling the pore characteristics and consequently the capacity and release characteristics of the ultimate product. For example, proper selection of the crosslinking means, the amount and type of crosslinking agent, and the amount and type of porogen are means of attaining such control. Certain polymerization conditions may also be varied to such effect, including temperature, degree of radiation where used, degree of agitation and any other factors affecting the rate of the polymerization reaction.

Crosslinking in the polymer formation is a major means of pore size control. Monomers which may be polymerized to produce crosslinked polymer beads in accordance with the present invention include polyethylenically unsaturated monomers, i.e., those having at least two sites of unsaturation, and monoethylenically unsaturated monomers in combination with one or more polyethylenically unsaturated monomers. In the latter case, the percentage of crosslinking may be controlled by balancing the relative amounts of monoethylenically unsaturated monomer and polyethylenically unsaturated monomer. The polymer beads of the present invention will have greater than 10% crosslinking, preferably from about 10% to about 80% crosslinking, and most preferably from about 20% to about 60% crosslinking. The percentage crosslinking is defined among those skilled in the art as the weight of polyethylenically unsaturated monomer or monomers divided by the total weight of monomer, including both polyethylenically unsaturated and monoethylenically unsaturated monomers.

Monoethylenically unsaturated monomers suitable for preparing polymer beads for the polymer delivery system include ethylene, propylene, isobutylene, diisobutylene, styrene, ethylvinylbenzene, vinylpyridine, vinyltoluene, and dicyclopentadiene; esters of acrylic and methacrylic acid, including the methyl, ethyl, propyl, isopropyl, butyl, sec-butyl, tert-butyl, amyl, hexyl, octyl, ethylhexyl, decyl, dodecyl, cyclohexyl, isobornyl, phenyl, benzyl, alkylphenyl, ethoxymethyl, ethoxyethyl, ethoxypropyl, propoxymethyl, propoxyethyl, propoxypropyl, ethoxyphenyl, ethoxybenzyl, and ethoxycyclohexyl esters; vinyl esters, including vinyl acetate, vinyl propionate, vinyl butyrate and vinyl laurate; vinyl ketones, including vinyl methyl ketone, vinyl ethyl ketone, vinyl isopropyl ketone, and methyl isopropenyl ketone; vinyl ethers, including vinyl methyl ether, vinyl ethyl ether, vinyl propyl ether, and vinyl isobutyl ether.

Polyethylenically unsaturated monomers which ordinarily act as though they have only one unsaturated group, such as isopropene, butadiene and chloroprene, may be used as part of the monoethylenically unsaturated monomer content.

Polyethylenically unsaturated crosslinking monomers suitable for preparing such polymer beads include diallyl phthalate, ethylene glycol diacrylate, ethylene glycol dimethacrylate, trimethylolpropanetrimethacrylate, divinylsulfone; polyvinyl and polyallyl ethers of ethylene glycol, of glycerol, of pentaerythritol, of diethyleneglycol, of monothio- and dithio-derivatives of glycols, and of resorcinol; divinylketone, divinylsulfide, allyl acrylate, diallyl maleate, diallyl fumarate, diallyl succinate, diallyl carbonate, diallyl malonate, diallyl oxalate, diallyl adipate, diallyl sebacate, divinyl sebacate, diallyl tartrate, diallyl silicate, triallyl tricarballylate, triallyl aconitate, triallyl citrate, triallyl phosphate, divinyl naphthalene, divinylbenzene, trivinylbenzene; alkyldivinylbenzenes having from 1 to 4 alkyl groups of 1 to 2 carbon atoms substituted on the benzene nucleus; alkyltrivinylbenzenes having 1 to 3 alkyl groups of 1 to 2 carbon atoms substituted on the benzene nucleus; trivinylnaphthalenes, and polyvinylanthracenes.

The preferred polymer bead of the present invention will be free from reactive groups which will interact with the porogen and the active ingredient which is ultimately incorporated in the composition. In particular, the beads should be free from reactive amino, hydroxyl, carboxylic, and other reactive functionalities. Such beads will not readily undergo unwanted reactions, will be stable over a wide pH range, will resist moderate oxidation and reduction, will be stable at higher temperatures, will resist attack by moisture, and will have a relatively long shelf life.

Particularly preferred polymer delivery systems of the present invention are formed by the copolymerization of styrene and divinylbenzene, vinyl stearate and divinylbenzene, 4-vinylpyridine and ethylene glycol dimethacrylate, or methylmethacrylate and ethylene glycol dimethacrylate. Usually, the monoethylenically unsaturated monomer will be present at from about 20% to 80% of the monomer mixture, with the polyethylenically unsaturated monomer forming the remainder of the mixture. Particularly preferred is the styrene-divinylbenzene polymeric bead which consists essentially of a hydrocarbon backbone with benzene rings and which is substantially completely free from reactive groups.

Once the microspheres are formed and dried, they are impregnated with the impregnant by contact absorption. As an option, the impregnant may be used in the form of a solution in a suitable organic solvent for purposes of decreasing viscosity and facilitating absorption. Examples of such solvents are liquid petrolatum, ether, petroleum ether, alcohols including methanol, ethanol and higher alcohols, aromatics including benzene and toluene, alkanes including pentane, hexane and heptane, ketones including acetone

and methyl ethyl ketone, chlorinated hydrocarbons including chloroform, carbon tetrachloride, methylene chloride and ethylene dichloride, acetates including ethyl acetate, and oils including isopropyl myristate, diisopropyl adipate and mineral oil. After absorption of the solution, the solvent can be evaporated or, if desired, retained inside the pores together with the impregnant. Other formulating materials, such as carriers or adjuvants such as fragrances, preservatives, antioxidants, and other emollients can also be present, and will be incorporated into and onto the beads together with the impregnants and any other materials present.

The impregnant, whether it be pure active ingredient, a mixture of active ingredients or a solution of active ingredient, will generally comprise between approximately 5% and approximately 65% of the total weight of the impregnated beads. When the active ingredient is a potent drug, it will generally be in the form of a dilute solution, and the weight percent of the active ingredient itself will range as low as 0.01% based on the total weight of the impregnated beads.

For topical application, the impregnated beads of the present invention may be used alone or in the form of fluid compositions or preparations similar to those commonly used for skin treatment, for example: gels, creams, lotions, ointments, sprays, powders, or oils. Appropriate vehicles for particular areas or methods of application will be readily apparent to those skilled in the art. When liquid vehicles are used (such as gels, creams, lotions, ointments or oils) and the impregnant is a solution of an active ingredient in a solvent, the solvent and vehicle must be immiscible so that outward diffusion of the active ingredient will not be accelerated by mutual diffusion between the solvent and vehicle. Appropriate combinations will therefore include the combination of a polar solvent and a nonpolar vehicle, and the combination of a nonpolar solvent and a polar vehicle.

The following are some of the considerations specific to various particular types of impregnants, plus examples of preparation and utility. The examples are offered solely for purposes of illustration. All parts and percentages are by weight, unless otherwise stated.

### 3.1 Topical Formulations for the Promotion of Hair Growth

For topical application, the impregnated beads of the present invention may be used alone or in the form of fluid compositions or preparations similar to those commonly used for skin treatment, for example: gels, creams, lotions, ointments, sprays, powders, or oils. Appropriate vehicles for particular impregnants or areas or methods of application will be readily apparent to those skilled in the art.

Minoxidil, a hair growth promotion agent, is a solid soluble in propylene glycol, and may only be incorporated into the beads by the two-step process.

#### 3.1.1 Example

A 2000 ml four-necked reaction flask equipped with a motorized stirrer, reflux condenser, thermometer, and nitrogen inlet were evacuated and purged with nitrogen. 800 parts of deionized water, 6.4 parts of gum arabic and 6.4 parts of a lignosulfonate available from Reed Lignins, Inc., under the trademark Marasperse N-22, were charged to the reaction flask. The mixture was stirred for about 30 minutes at about 50 °C until the dispersants (gum arabic and lignosulfate) dissolved to form an aqueous phase.

To this mixture was added a freshly prepared solution of 85.6 parts of styrene (99.8% purity), 102.3 parts of commercial divinylbenzene (55.6% divinyl benzene, 42.3% ethylvinylbenzene), 5.3 parts of benzoyl peroxide (70% active ingredient and 30% water), and 187.9 parts of toluene to serve as a porogen. The aqueous phase and organic solution were agitated by stirring at a rate adjusted to give a plurality of droplets having an average droplet diameter of about 10-60 µm, as determined by visual observation of a sample of the droplets with an optical microscope (400X) with the droplets being stabilized by the dispersants. This rate was approximately 1200 rpm. The reaction mixture was heated to about 78 °C and constantly stirred while a slow stream of nitrogen is passed through the reaction vessel, thus forming porous beads of crosslinked styrene/divinylbenzene copolymer having heptane entrapped within the network of pores. The mixture was stirred another 22 hours at 78 °C and allowed to cool to room temperature. The mixture was then diluted with 200 parts of water, and the porous polymeric beads were removed from the reaction flask by filtration. The filtered beads were washed initially three times with one liter portions of deionized water to remove the dispersants, followed by several washes of isopropanol/acetone mixture (7:3, respectively, by weight) to remove any residual, unreacted monomer and the heptane used as the porogen during polymerization. The beads were filtered and then dried at 65 °C in vacuo.

The calculated or theoretical crosslinking density of the purified beads was 30%. This density is calculated by multiplying the weight of divinylbenzene (102.3 g) by the purity of the divinylbenzene (.55) to

get the actual weight of pure divinylbenzene which is then divided by the total weight of monomer (102.3 g + 85.6 g).

The surface area of a sample of the purified beads was determined by the B.E.T. method to be 1.8 meters$^2$/gram. The B.E.T. method is described in detail in Brunauer, S. Emmet, P.H., and Teller, E., J. Am. Chem. Soc., 60, 309-16 (1938).

The particle size of the beads was determined by an optical microscope to be 60 µm or less with an average approximate particle size diameter of about 10 µm.

Minoxidil was obtained from Minoxidil 10 mg tablets (Upjohn, Loniten Tablet, 10 mg) by an ethanol extraction in which Minoxidil tablets were first ground into a powder. 3.4 grams of the ground powder were then mixed with 15 ml of ethanol (95% ethanol and 5% ispropyl alcohol) and the resultant mixture was stirred for 10 minutes. The mixed solution produced by stirring was then filtered and the ethanol was evaporated. 0.35 g of white Minoxidil solid, which shall be referred to as CH 215M, was obtained.

In a first example of preparation of polymeric beads having a solution of an active ingredient useful for promoting hair growth dissolved in a percutaneous absorption enhancer held within a network of pores of the polymeric beads, 10.6 mg of the white Minoxidil solid CH 215M was dissolved in 2 grams of a 50:50 solution of propylene carbonate/ethanol. 1.5 grams of the polymeric beads CH 215 was then mixed into the solution until the product is homogeneous. The Minoxidil content in the resulting product, which shall be referred to as CH 215 A-3, was 3 mg/g. In a second example, 1.2 parts of the white Minoxidil solid CH 215M, was added to 32.1 parts of propylene glycol and stirred in a flask at 45°C until the white Minoxidil solid CH 215M was completely dissolved. Thereafter, 61.7 parts of polymeric beads (CH 215) were added to the solution which was mixed until the product is homogeneous. The Minoxidil content in the resulting product was is 12 mg/g.

A composition useful in the method of the present invention is then prepared by adding 9.09 parts of CH 215 A-3 to 90.01 parts of a medium comprised of petroleum jelly. The product was then mixed until homogeneous. The Minoxidil content in the resulting product was 1.1 mg/g. This product could then be rubbed into portions of the skin and scalp to enhance hair growth.

While the above description has described the preparation of a composition useful in the method of the present invention to promote hair growth, it should be noted that a product containing higher concentrations of Minoxidil could be used to achieve the known cardiovascular effects of Minoxidil. Thus, for example, such a product could be rubbed onto the skin to provide a topical application of Minoxidil, as opposed to an oral dosage, for use in controlling hypertension.

### 3.2 Topical Compositions used in the Treatment of Acne

Substances active as acne treatment agents which can be used in the present invention comprise benzoyl peroxide, optionally together with salicylic acid. Benzoyl peroxide cannot be used as a porogen, so the two-step process is necessary.

#### 3.2.1 Example

A 2000 ml four-necked reaction flask equipped with a motorized stirrer, reflux condenser, thermometer, and nitrogen inlet was evacuated and purged with nitrogen. 800 parts of deionized water, 6.4 parts of gum arabic and 6.4 parts of a sodium-based lignosulfonate available from Reed Lignins, Inc., under the trademark Marasperse N-22, were charged to the reaction flask. The mixture was heated, with stirring, in an oil bath at about 50°C until the dispersants (gum arabic and lignosulfate) dissolved to form an aqueous phase.

To this mixture there was then added a freshly prepared solution of 102.3 parts of styrene (99.8% purity), 85.6 parts of commercial divinylbenzene (55.6% divinyl benzene, 42.3% ethylvinylbenzene), 5.3 parts of benzoyl peroxide (70% active ingredient and 30% water), and 130 parts of heptane. The aqueous phase and organic solution were agitated by stirring at a rate adjusted to give a plurality of droplets having an average droplet diameter of about 10-60 µm, as determined by visual observation of a sample of the droplets with an optical microscope (400X) with the droplets being stabilized by the dispersants. This rate is approximately 1200 rpm. The reaction mixture was then heated to about 80°C and maintained at that temperature for about 20 hours, at the previously adjusted stirring rate, to form porous beads of crosslinked styrene/divinylbenzene copolymer having heptane entrapped within the network of pores. The mixture was then cooled, diluted with 200 parts of water, and the porous polymeric beads were removed from the reaction flask by filtration. The filtered beads were washed initially three times with one liter portions of deionized water to remove the dispersants, followed by three washes with 0.6 liter portions of isopropanol:

acetone mixture (7:3, respectively, by weight) to remove any residual, unreacted monomer and the heptane used as the porogen during polymerization. The beads were then dried in an oven at 80-100°C for eight hours.

The average particle diameter of these beads was 25 µm, as measured by a Sedimentation Micromeritics Microsizer 5300, an instrument available from Micromeritics Instrument Company, Norcross, Georgia. The particle diameter determination method is described in detail in the "Microsizer 5300 Particle Size Analyzer Instruction Manual" (1984) associated with the instrument.

The calculated or theoretical crosslinking density of the purified beads is 25%. This density is calculated by multiplying the weight of divinylbenzene (85.6 parts) by the purity of the divinylbenzene (55.6%) to get the actual weight of pure divinylbenzene which is then divided by the total weight of monomer (85.6 parts + 102.3 parts) and multiplied by 100.

The surface area of a sample of the purified beads was determined by the B.E.T. nitrogen multipoint analysis to be 91.2 $m^2$/g while the pore volume was determined by the mercury intrusion method to be 1.0 ml/g. The B.E.T. method is described in detail in Brunauer, S., Emmet, P.H., and Teller, E., J. Am. Chem. Soc., 60, 309-16 (1938). The mercury intrusion method is described in detail in "Advanced Experimental Techniques in Powder Metallurgy," pages 225-252, (Plenum Press, 1970).

### 3.2.2 and 3.2.3 Examples

By repeating the procedure of Example 3.2.1 in every essential detail, except for the weights of monomers and porogen employed, porous crosslinked polymer beads were obtained having the following characteristics listed in Table 3.2.1:

TABLE 3.2.1

| Example | Ratio Of Parts Styrene/Divinyl-Benzene/Porogen | Calculated Crosslnkng Density, % | Avg. Particle Diam, µm | Surface Area $m^2$/g | Pore Volume ml/g |
|---|---|---|---|---|---|
| 2 | 89/100/180 (porogen = mineral oil) | 29 | 25 | 75 | 1.36 |
| 3 | 85.6/102.3/188 (porogen = toluene) | 30 | 25 | 1.8 | 0.04 |

### 3.2.4 Example

A 10 part portion of the macroporous crosslinked polymer beads prepared as described in each of Examples 3.2.1-3.2.3 above was mixed at room temperature with 16 parts of a 12.5% solution of benzoyl peroxide in acetone, and the resulting suspensions were hand-stirred for a few minutes. The thus-obtained homogeneous wet powders were washed three times with 30 ml portions of deionized water in a funnel, then air dried at room temperature for 20 hours. The benzoyl peroxide contents entrapped within these beads' macropores as determined by titration with iodine in isopropanol and based on the total weight of beads and entrapped benzoyl peroxide, were as follows:

TABLE 3.2.2

| Beads Of Example | Wt Of Water, % | Wt. Of Benzoyl Peroxide, % |
|---|---|---|
| 1 | 1 | 12.2 |
| 2 | 1 | 12.1 |
| 3 | 8 | 9.6 |

### 3.2.5 Example

Two 10 part portions of the macroporous crosslinked polymer beads prepared as described in Example 3.2.1 above were mixed at room temperature with a 14 part portion of a 12.5% solution of benzoyl peroxide

in acetone and a 10 part portion of a 20% solution of salicylic acid in acetone, respectively. The resulting wet powders were hand-stirred until homogeneous, then air-dried at room temperature for 20 hours. Their respective contents of benzoyl peroxide and salicylic acid, as determined by titration with iodine and dilute aqueous sodium hydroxide, respectively, and based on the total weight of beads and entrapped benzoyl peroxide and salicylic acid, were:

| | |
|---|---|
| Benzoyl peroxide | 11.3% |
| Salicylic acid | 16% |

The two lots of beads were then commingled to provide a therapeutic delivery system for topically applying benzoyl peroxide and salicylic acid together to the skin.

### 3.3 Vitamins and Vitamin Derivatives

Any vitamin, vitamin derivative or vitamin-containing substance which can be applied topically to human or animal skin can be absorbed in the above-described minute polymer beads to form the novel, macroporous, high capacity topical delivery systems of this invention. The chemical nature of each species will establish whether or not it can be used in the one-step procedure. Retinoids, in particular, can in general only be used in the two-step procedure.

#### 3.3.1 Example

A 2000 ml four-necked reaction flask equipped with a motorized stirrer, reflux condenser, thermometer, and nitrogen inlet was evacuated and purged with nitrogen. 800 parts of deionized water, 6.4 parts of gum arabic and 6.4 parts of a sodium-based lignosulfonate available from Reed Lignins, Inc., under the trademark Marasperse N-22, were charged to the reaction flask. The mixture was heated, with stirring, in an oil bath at about 50° C until the dispersants (gum arabic and lignosulfate) dissolved to form an aqueous phase.

To this mixture was then added a freshly prepared solution of 90.5 parts of styrene (99.8% purity), 55 parts of commercial divinylbenzene (55.6% divinyl benzene, 42.3% ethylvinylbenzene), 2 parts benzoyl peroxide (70% active ingredient and 30% water), and 69.4 parts of heptane (porogen). The aqueous phase and organic solution were agitated by stirring at a rate adjusted to give a plurality of droplets having an average droplet diameter of about 10-60 µm, as determined by visual observation of a sample of the droplets with an optical microscope (400X) with the droplets being stabilized by the dispersants. This rate was approximately 1200 rpm. The reaction mixture was then heated to about 80° C and maintained at that temperature for about 12 hours, at the previously adjusted stirring rate, to form porous beads of crosslinked styrene/divinylbenzene copolymer having toluene entrapped within the network of pores. The mixture was then cooled and the porous polymeric beads are removed from the reaction flask by filtration. The filtered beads were washed initially three times with one liter portions of deionized water to remove the dispersants, followed by three washes with 0.6 liter portions of isopropanol to remove any residual, unreacted monomer and the toluene used as the porogen during polymerization. The beads were then dried in an oven at 80° C for eight hours.

The average particle diameter of these beads, which are white and opaque in appearance, indicating their macroporosity, was less than 35 microns, as measured by a mercury intrusion porosimeter or by optical microscopy.

The calculated or theoretical crosslinking density of the purified beads was 21.01%. This density was calculated by multiplying the weight of divinylbenzene (55 parts) by the purity of the divinylbenzene (0.556) to get the actual weight of pure divinylbenzene which was then divided by the total weight of monomer (90.5 parts + 55 parts) and multiplied by 100.

The surface area of a sample of the purified beads was determined by the B.E.T. method to be 36.41 $meters^2/gram$ while the pore volume was determined by nitrogen adsorption isotherm to be 0.206 ml/gram. The B.E.T. method is described in detail in Brunauer, S. Emmet, P.H., and Teller, E., J. Am. Chem. Soc., 60, 309-16 (1938). The nitrogen adsorption isotherm method is described in detail in Barrett, E.P., Joyner, L.G. and Helenda, P.P., J. Am. Chem. Soc., 73, 373-80 (1951).

### 3.3.2 Example

The procedure of Example 3.4.1 was repeated in every essential detail, except for the following: 800 parts of deionized water were used to dissolve 5.6 parts of gum arabic and 5.6 parts of Marasperse N-22 at about 23°C; 105 parts of styrene and 9.5 parts of divinylbenzene were used; 2.8 parts of benzoyl peroxide (70% active ingredient and 30% water) and 120 parts of heptane were employed during polymerization and stirring was adjusted to give an average droplet diameter of below about 50 µm (rate approximately 800-1600 rpm); three 300 ml portions of isopropanol were used to wash the beads. The macroporous crosslinked polymer beads obtained had the following characteristics:

| | |
|---|---|
| Calculated Crosslinking Density, %: | 26.4 |
| Average Particle Diameter, f: | 25 |
| Surface Area, $m^2/g$: | 85.9 |
| Pore Volume, ml/g: | 0.44 |

### 3.3.3 Example

A two liter four-necked reaction flask equipped as described in Example 3.4.1 was evacuated and purged with nitrogen. An aqueous phase made up of 600 parts of deionized water, 6.0 parts of gum arabic and 6.0 parts of Marasperse N-22 was added to the flask, and an organic solution made up of 72.0 parts of methyl methacrylate, 78.0 parts of ethylene glycol dimethacrylate, 2.0 parts of benzoyl peroxide (70% active ingredient and 30% water) and 108.4 parts of toluene was dispersed in the aqueous phase with strong agitation (stirrer speed approximately 1000 rpm) to obtain plurality of droplets having an average droplet diameter of below about 50 µm, as determined by visual observation of a sample of the droplets with an optical microscope (400 x), with the droplets being stabilized by the dispersants.

The reaction mixture was then heated to 80°C and maintained at that temperature for 12 hours while maintaining a nitrogen flow of 6 ml/minute, to form porous beads of crosslinked methyl methacrylate/ethylene glycol dimethacrylate copolymer having toluene entrapped within the pores. The reaction mixture was then cooled and the beads were collected by filtration, washed three times with 1000 part portions of water, then three times with 1000 part portions of isopropanol, and then dried at 80°C for about 8 hours.

The calculated or theoretical crosslinking density of the purified beads was 52%, and was calculated by dividing the weight of ethylene glycol dimethacrylate (78.0 parts) by the total weight of monomer (72.0 parts + 78.0 parts) and then multiplying by 100.

The surface area of a sample of the purified beads is 96 $meters^2/gram$ and the pore volume is 0.36 ml/gram, determined as described in Example 3.4.1 above.

### 3.3.4 Example

The procedure of Example 3.3.3 was again repeated in every essential detail except for the following: 400 parts of deionized water were used to dissolve 4.0 parts of gum arabic and 4.0 parts of Marasperse N-22; 70 parts of methyl methacrylate and 30 parts of ethylene glycol dimethacrylate were used; 1.0 part of lauroyl peroxide and 69.4 parts of toluene were employed during polymerization; the reaction was conducted at 85°C for 12 hours. The resulting polymer beads were collected and washed with three 1000 ml portions of deionized water followed by three 1000 ml portions of isopropanol, and then dried at 80°C for about 8 hours. The macroporous crosslinked polymer beads obtained had the following characteristics:

| | |
|---|---|
| Calculated Crosslinking Density, %: | 30 |
| Average Particle Diamter, µm: | 30 |
| Surface Area, $M^2/g$: | 12.54 |
| Pore Volume, ml/g: | 0.170 |

### 3.3.5-3.3.7 Examples

By repeating the procedure of Example 3.3.4 in every essential detail except for the weights of monomers and solvents employed, the macroporous cross-linked polymer beads described in Table 3.3.1 below were obtained.

TABLE 3.3.1

| Example | Methyl Methacrylate, g | Ethylene Glycol Dimethacrylate, g | Porogen Toluene, g | Calculated Crosslinking Density, % | Average Particle Diameter, µm | Surface Area $m^2/g$ | Pore Volume, ml/g |
|---|---|---|---|---|---|---|---|
| 5 | 20.0 | 80.0 | 85.6 | 80.0 | 30 | 301.93 | 0.553 |
| 6 | 40.0 | 60.0 | 69.4 | 60.0 | 25 | 95.07 | 0.368 |
| 7 | 80.0 | 20.0 | 86.7 | 20.0 | 40 | 0.72 | 0.044 |

3.3.8 Example

A 1.0 part portion of the macroporous cross-linked polymer beads prepared as described in Example 3.3.3 above was mixed at room temperature with 4.0 parts of ethanol in a glass beaker with an agitator. Vitamin A (1.0 parts) is then added slowly, with stirring, and the resulting suspension is stirred for about five minutes. The solvent is then allowed to evaporate to dryness in a fume hood at room temperature for 2 days.

Methyl methacrylate/ethylene glycol dimethacrylate macroporous crosslinked polymer beads containing 50% of vitamin A are obtained.

3.3.9 Example

A 2000 ml four-necked reaction flask equipped with a motorized stirrer, reflux condenser, thermometer, and nitrogen inlet was evacuated and purged with nitrogen. 1200 parts of deionized water, 9.6 parts of gum arabic and 9.6 parts of a sodium-based lignosulfonate available from Reed Lignins, Inc., under the trademark Marasperse N-22, were charged to the reaction flask. The mixture was heated, with stirring, in an oil bath at about 50° C until the dispersants (gum arabic and lignosulfate) dissolved to form an aqueous phase.

To this mixture there was then added a freshly prepared solution of 90.5 parts of styrene (99.8% purity), 55 parts of commercial divinylbenzene (55.6% divinyl benzene, 42.3% ethylvinylbenzene), 2 parts of benzoyl peroxide (70% active ingredient and 30% water), and 69.4 parts of heptane. The aqueous phase and organic solution were agitated by stirring at a rate adjusted to give a plurality of droplets having an average droplet diameter of about 10-60 µm, as determined by visual observation of a sample of the droplets with an optical microscope (400X) with the droplets being stabilized by the dispersants. This rate is approximately 1200 rpm. The reaction mixture was then heated to about 85° C and maintained at that temperature for about 12 hours, at the previously adjusted stirring rate, to form porous beads of crosslinked styrene/divinylbenzene copolymer having heptane entrapped within the network of pores. The mixture was then cooled and the porous polymeric beads were removed from the reaction flask by filtration. The filtered beads were washed initially three times with one liter portions of deionized water to remove the dispersants, followed by three washes with one-liter portions of isopropanol to remove any residual, unreacted monomer and porogen. The beads were then dried in an oven at 80° C for eight hours. The average particle diameter of these beads, which were white and opaque in appearance, indicating their macroporosity, was less than 35 µm, as measured by a Sedimentation Micromeritics Microsizer 5300, an instrument available from Micromeritics Instrument Company, Norcross, Georgia. The particle diameter determination method is described in detail in the "Microsizer 5300 Particle Size Analyzer Instruction Manual" (1984) associated with the instrument.

The calculated or theoretical crosslinking density of the purified beads is 21.01%. This density is calculated by multiplying the weight of divinylbenzene (55 parts) by the purity of the divinylbenzene (55.6%) to get the actual weight of pure divinylbenzene which is then divided by the total weight of monomer (90.5 parts + 55 parts) and multiplied by 100.

The surface area of a sample of the purified beads was determined by the B.E.T. nitrogen multipoint analysis to be 36.41 $m^2$/g while the pore volume was determined by the mercury intrusion method to be 0.206 ml/g. The B.E.T. method is described in detail in Brunauer, S., Emmet, P.H., and Teller, E., J. Am. Chem. Soc., 60, 309-16 (1938). The mercury intrusion method is described in detail in "Advanced Experimental Techniques in Powder Metallurgy," pages 225-252, (Plenum Press, 1970).

3.3.10 Example

The procedure of Example 3.3.9 was repeated in every essential detail, except for the following: 750 parts of deionized water were used to dissolve 7.0 parts of gum arabic and 7.0 parts of Marasperse N-22 at about 23° C; 75 grams of styrene and 75 grams of divinylbenzene were used; 1.0 part of benzoyl peroxide and 65.03 parts of heptane were employed during polymerization and stirring was adjusted to give an average droplet diameter of below about 50 µm (rate approximately 800-1600 rpm); and three 300-ml portions of isopropanol were used to wash the beads. The macroporous crosslinked polymer beads obtained had the following characteristics:

| | |
|---|---|
| Calculated Crosslinking Density, %: | 27.8 |
| Average Particle Diameter, µm: | 25 |
| Surface Area, $m^2/g$: | 59.43 |
| Pore Volume, ml/g: | 0.377 |

### 3.3.11 Example

A two-liter four-necked reaction flask equipped as described in Example 3.3.9 was evacuated and purged with nitrogen. An aqueous phase made up of 600 parts of deionized water, 6.0 parts of gum arabic and 6.0 parts of Marasperse N-22 was added to the flask, and an organic solution made up of 72.0 parts of methyl methacrylate, 78.0 parts of ethylene glycol dimethacrylate, 2.0 parts of a 70% aqueous solution of benzoyl peroxide and 108.4 parts of toluene was dispersed in the aqueous phase with strong agitation (stirrer speed approximately 1000 rpm) to obtain a plurality of droplets having an average droplet diameter of below about 50 µm, as determined by visual observation of a sample of the droplets with an optical microscope (400X), the droplets being stabilized by the dispersants.

The reaction mixture was then heated to 85°C and maintained at that temperature for 12 hours while maintaining a nitrogen flow of 2 ml/minute, to form porous beads of crosslinked methyl methacrylate/ethylene glycol dimethacrylate copolymer having toluene entrapped within the pores. The reaction mixture was then cooled and the beads collected by filtration, washed three times with 1000 part portions of water, and three times with 1000 part portions of isopropanol, then dried in air at 80°C for about 8 hours.

The calculated or theoretical crosslinking density of the purified beads is 52%, and was calculated by dividing the weight of ethylene glycol dimethacrylate (78.0 parts) by the total weight of monomer (150 parts), and then multiplying by 100.

The surface area of a sample of the purified beads is 96 $m^2/g$ and the pore volume is 0.36 ml/g, determined as described in Example 1 above.

### 3.3.12-3.3.13 Examples

By repeating the procedure of Example 3.3.11 in every essential detail except for the weights of monomers employed, the macroporous crosslinked polymer beads described in the following table were obtained.

| | Example 3.3.12 | Example 3.3.13 |
|---|---|---|
| Methyl Methacrylate, g | 48.0 | 40.0 |
| Ethylene Glycol Dimethacrylate, g | 52.0 | 60.0 |
| Calculated Cross-linking Density, % | 51.0 | 58.8 |
| Average Particle Diameter, microns | 30 | 25 |
| Surface Area, $m^2/g$ | 92.70 | 95.07 |
| Pore Volume, ml/g | 0.366 | 0.368 |

### 3.3.14 Example

A 6-part portion of the macroporous crosslinked polymer beads prepared in Example 3.3.9 above is mixed at room temperature with 4 parts of retinoic acid (all-trans-form) dissolved in 10 ml of ethanol. The resulting suspension is hand-stirred for a few minutes, and the solvent in then allowed to evaporate to dryness in a fume hood at room temperature. The beads are calculated to contain 40% of retinoic acid retained within the pores.

### 3.3.15 Example

A 10-part portion of the macroporous cross-linked polymer beads prepared in each of Examples 3.3.10 through 3.3.13 is mixed at room temperature with 4 parts of retinoic acid (all-trans-form) dissolved in a solution of 8 parts of isopropyl myristate and 8 parts of isopropanol. The resulting suspension is hand-stirred and the solvent is then allowed to evaporate. The beads are calculated to contain 18.2% of retinoic acid and 36.4% of isopropyl myristate retained within the pores.

**3.4 Epidermal Lipid Replacement Substances**

These comprise squalene and/or squalane.

**3.4.1 Example**

A 2000 ml four-necked reaction flask equipped with a motorized stirrer, reflux condenser, thermometer, and nitrogen inlet was evacuated and purged with nitrogen. 800 parts of deionized water, 6.4 parts of gum arabic and 6.4 parts of a sodium-based lignosulfonate available from Reed Lignins, Inc., under the trademark Marasperse N-22, were charged to the reaction flask. The mixture was heated, with stirring, in an oil bath at about 50°C until the dispersants (gum arabic and lignosulfate) dissolved to form an aqueous phase.

To this mixture there was then added a freshly prepared solution of 102.3 parts of styrene (99.8% purity), 85.6 parts of commercial divinylbenzene (55.6% divinyl benzene, 42.3% ethylvinylbenzene), 5.3 parts of benzoyl peroxide (70% active ingredient and 30% water), and 130 parts of heptane. The aqueous phase and organic solution were agitated by stirring at a rate adjusted to give a plurality of droplets having an average droplet diameter of about 10-60 µm, as determined by visual observation of a sample of the droplets with an optical microscope (400X) with the droplets being stabilized by the dispersants. This rate is approximately 1200 rpm. The reaction mixture was then heated to about 80°C and maintained at that temperature for about 20 hours, at the previously adjusted stirring rate, to form porous beads of crosslinked styrene/divinylbenzene copolymer having heptane entrapped within the network of pores. The mixture was then cooled, diluted with 200 parts of water, and the porous polymeric beads were removed from the reaction flask by filtration. The filtered beads were washed initially three times with one liter portions of deionized water to remove the dispersants, followed by three washes with 0.6 liter portions of isopropanol:acetone mixture (7:3, respectively, by weight) to remove any residual, unreacted monomer and the heptane used as the porogen during polymerization. The beads were then dried in an oven at 80-100°C for eight hours. The average particle diameter of these beads was 25 µm, as measured by a Sedimentation Micromeritics Microsizer 5300, an instrument available from Micromeritics Instrument Company, Norcross, Georgia. The particle diameter determination method is described in detail in the "Microsizer 5300 Particle Size Analyzer Instruction Manual" (1984) associated with the instrument.

The calculated or theoretical crosslinking density of the purified beads is 25%. This density is calculated by multiplying the weight of divinylbenzene (85.6 parts) by the purity of the divinylbenzene (55.6%) to get the actual weight of pure divinylbenzene which is then divided by the total weight of monomer (85.6 parts + 102.3 parts) and multiplied by 100.

The surface area of a sample of the purified beads was determined by the B.E.T. nitrogen multipoint analysis to be 91.2 $m^2/g$ while the pore volume was determined by the mercury intrusion method to be 1.0 ml/g. The B.E.T. method is described in detail in Brunauer, S., Emmet, P.H., and Teller, E., J. Am. Chem. Soc., 60, 309-16 (1938). The mercury intrusion method is described in detail in "Advanced Experimental Techniques in Powder Metallurgy," pages 225-252, (Plenum Press, 1970).

**3.4.2 Example**

A two-liter four-necked reaction flask equipped as described in Example 3.6.1 was evacuated and purged with nitrogen. An aqueous phase made up of 600 parts of deionized water, 6.0 parts of gum arabic and 6.0 parts of Marasperse N-22 was added to the flask, and an organic solution made up of 72.0 parts of methyl methacrylate, 78.0 parts of ethylene glycol dimethacrylate, 2.0 parts of benzoyl peroxide and 108.4 parts of toluene was dispersed in the aqueous phase with strong agitation (stirrer speed approximately 1000 rpm) to obtain a plurality of droplets having an average droplet diameter of below about 50 µm, as determined by visual observation of a sample of the droplets with an optical microscope (400X), the droplets being stabilized by the dispersants.

The reaction mixture was then heated to 85°C and maintained at that temperature for 12 hours while maintaining a nitrogen flow of 2 ml/minute, to form porous beads of crosslinked methyl methacrylate/ethylene glycol dimethacrylate copolymer having toluene entrapped within the pores. The reaction mixture was then cooled and the beads collected by filtration, washed three times with 1000 parts of water, and three times with 1000 parts of isopropanol, then dried in air at 80°C for about 8 hours.

The calculated or theoretical crosslinking density of the purified beads is 52%, and was calculated by dividing the weight of ethylene glycol dimethacrylate (78.0 parts) by the total weight of monomer (150 parts), and then multiplying by 100.

The surface area of a sample of the purified beads is 96 $m^2$/g and the pore volume is 0.36 ml/g, determined as described in Example 1 above.

**3.4.3 Example**

Ten-part portions of preformed dry polymer beads from Examples 3.4.1 and 3.4.2 were each mixed at room temperature with a ten-part portion of an 67% solution of squalane in hexane. The resulting suspensions were hand-stirred for a few minutes. The hexane was then allowed to evaporate from the resulting wet powders at room temperature. The resulting beads contained 40% squalane entrapped within their macropores.

**3.4.4 Example**

The procedure of Example 3.4.3 was repeated in every detail except for the following: two ten-part portions of the preformed beads prepared as described in each of Examples 3.4.1 and 3.4.2 were used, with one of the portions of the beads from each example being admixed with a ten-part portion of an 67% solution of squalene in hexane, and with the other two portions of beads being admixed with ten-part portions of an 67% solution of 50:50 mixture of squalene and squalane in hexane. In all four cases, the beads contained 40% of the absorbed, entrapped impregnant or impregnant mixture within their micropores.

Some further examples of syntheses of beads suitable for receiving impregnants will now be given.

**3.5.1 Example**

A two liter four-necked reaction flask equipped as described in Example 3.1.1 was evacuated and purged with nitrogen. An aqueous phase made up of 800 parts of deionized water, 8 parts of gum arabic and 8 parts of Marasperse N-22 was added to the flask, and an organic solution made up of 100 parts of methyl methacrylate, 100 parts of ethylene glycol dimethacrylate, 10 parts of butyl methacrylate, 2 parts of lauroyl peroxide and 173 parts of toluene was dispersed in the aqueous phase with strong agitation (stirrer speed approximately 1000 rpm) to obtain a plurality of droplets having an average droplet diameter of below about 50 µm, as determined by visual observation of a sample of the droplets being stabilized by the dispersants.

The reaction mixture was then heated to 80 °C and maintained at that temperature for 6 hours while maintaining a nitrogen flow of 1 ml/minute, to form porous beads of crosslinked methyl methacrylate/butyl methacrylate/ethylene glycol dimethacrylate terpolymer having toluene entrapped within the pores. The reaction mixture was then cooled and the beads were collected by filtration, washed three times with 1000 parts of water, and three times with 1000 parts of isopropanol, and then dried in air at room temperature.

The calculated or theoretical crosslinking density of the purified beads was 47.6%, and was calculated by dividing the weight of ethylene glycol dimethacrylate (100 parts) by the total weight of monomer 210 parts + (100 parts + 110 parts) and then multiplying by 100.

The surface area of a sample of the purified beads was 52 meters$^2$/gram and the pore volume was 0.4 ml/gram, determined as described in Example I above.

**3.5.2-3.5.5 Examples**

By repeating the procedure of Example 3.5.1 in every essential detail except for the weights of monomers employed and, in one case, the porogen used, the macroporous crosslinked polymer beads described in Table 3.5.1 below were obtained.

TABLE 3.5.1

| Example | Methyl Methacrylate | Ethylene Glycol Dimethacrylate | Porogen Parts | Calculated Crosslinking Density | Average Particle Diameter, μm | Surface Area $m^2/g$ | Pore Volume ml/g |
|---|---|---|---|---|---|---|---|
| 3 | 100 | 100 | 173[1] | 50% | 25 | 73.8 | 0.405 |
| 4 | 150 | 150 | 450[1] | 50% | 15 | 95.8 | 0.508 |
| 5 | 100 | 100 | 200[1] | 50% | 25 | 84.0 | 0.38 |
| 6 | 210 | 90 | 200[2] | 30% | 15 | 2.34 | 0.58 |

1 Toluene
2 Heptane

## Claims

**1.** A topical composition comprising solid particles containing a substantially continuous non-collapsible network of pores open to the exterior of said particles, and an impregnant retained inside said pores,

wherein said impregnant is optionally in a solvent and comprises one or more of: benzoyl peroxide; retinol, 3-dihydroretinol, retinoic acids, retinoic aldehydes, retinoic acid esters, and aromatic derivatives thereof; minoxidil; squalane; and squalene; prepared by:

(a) forming said particles by suspension polymerisation in the presence of a porogen so that porous particles with the porogen in the pores are formed;

(b) extracting the porogen from the pores; and

(c) impregnating the pores with said impregnant, and wherein said solid particles are substantially spherical in shape, are formed from a cross-linked polymer, having an average diameter of about 10 microns to about 40 µm, having a total pore volume of about 0.1 ml/g to about 2.0 ml/g, having a surface area of about 20 $m^2$/g to about 200 $m^2$/g, and having an average pore diameter of about 0.003 to about 1.0 µm.

**2.** A topical composition in accordance with claim 1 in which said impregnant is in a solvent selected from ethers, alcohols, aromatics and alkanes.

**3.** A topical composition in accordance with claim 1 or 2 in which said solid particles are formed of a copolymer of styrene and divinylbenzene; or of methyl methacrylate and ethylene glycol dimethacrylate or of 4-vinylpyridine and ethylene glycol dimethacrylate.

**4.** A topical composition in accordance with any preceding claim in which said solid particles are combined with a vehicle to form a composition selected from fluid, semi-solid and solid compositions.

**5.** A topical composition in accordance with any preceding claim in which said impregnant comprises from about 5% to about 65% thereof.

**6.** A method for preparing a topical composition according to any of claims 1 to 5 comprising:

(a) forming solid particles containing a substantially continuous network of pores open to the exterior of said particles; and

(b) impregnating said pores of said solid particles with an impregnant.

**7.** A method in accordance with claim 6 in which step (a) comprises:

(i) combining a monomer composition with a porogen which is a substantially water-immiscible liquid species which is inert with respect to said monomer composition to form a homogeneous, substantially water-immiscible liquid solution;

(ii) suspending said liquid solution in an aqueous solution to form a dispersion;

(iii) polymerizing said monomer composition in said dispersion to form solid beads containing said water-immiscible liquid chemical species;

(iv) recovering said solid beads from said dispersion; and (v) extracting said water-immiscible liquid chemical species from said solid beads.

**8.** A method for preparing a topical composition comprising solid particles containing a substantially continuous non-collapsible network of pores open to the exterior of said particles, and an impregnant retained inside said pores; said method comprising:

(a) forming said particles by suspension polymerisation in the presence of a porogen so that porous particles with the porogen in the pores are formed, said particles being substantially spherical in shape, formed from a cross-linked polymer, and having an average diameter of about 10 µm to about 40 µm, a total pore volume of about 0.1 ml/g to about 2.0 ml/g, a surface area of about 20 $m^2$/g to about 200 $m^2$/g, and an average pore diameter of about 0.003 to about 1.0 µm;

(b) extracting the porogen from the pores;and

(c) impregnating the pores with said impregnant and wherein said impregnant is optionally in a solvent and comprises one or more of benzoyl peroxide; retinol, 3-dihydroretinol, retinoic acids, retionoic aldehydes, retinoic acid esters, and aromatic derivatives thereof; minoxidil; squalane; and squalene.

**9.** A method for cosmetic treatment of an epidermal region, said method comprising applying to said epidermal region a topical composition according to any of claims 1 to 5, said impregnant comprising a cosmetic treatment agent.

## Patentansprüche

1. Topische Zusammensetzung, die feste Teilchen umfaßt, die ein im wesentlichen kontinuierliches, unzusammendrückbares Netzwerk aus Poren, die zur Außenseite der genannten Teilchen offen sind, und ein innerhalb der genannten Poren gehaltenes Imprägniermittel enthalten, worin das genannte Imprägniermittel wahlweise in einem Lösungsmittel vorliegt und eines oder mehrere aus Benzoylperoxid, Retinol, 3-Dihydroretinol, Retinsäuren, Retinaldehyden, Retinsäureestern und aromatischen Derivaten davon, Minoxidil, Squalan und Squalen enthält; hergestellt durch:
   (a) Bilden der genannten Teilchen durch Suspensionspolymerisation in der Gegenwart eines Porogens, sodaß poröse Teilchen mit dem Porogen in den Poren gebildet werden;
   (b) Extrahieren des Porogens aus den Poren; und
   (c) Imprägnieren der Poren mit dem genannten Imprägniermittel, und worin die genannten festen Teilchen im wesentlichen Kugelgestalt aufweisen, aus vernetztem Polymer gebildet sind, einen durchschnittlichen Durchmesser von etwa 10 µm bis etwa 40 µm aufweisen, ein Gesamtporenvolumen von etwa 0,1 ml/g bis etwa 2,0 ml/g aufweisen, eine Oberfläche von etwa 20 $m^2/g$ bis etwa 200 $m^2/g$ aufweisen und einen durchschnittlichen Porendurchmesser von etwa 0,003 bis etwa 1,0 µm aufweisen.

2. Topische Zusammensetzung nach Anspruch 1, bei der das genannte Imprägniermittel in einem Lösungsmittel vorliegt, das aus Äthern, Alkoholen, Aromaten und Alkanen ausgewählt ist.

3. Topische Zusammensetzung nach Anspruch 1 oder 2, bei der die genannten festen Teilchen aus einem Copolymer von Styrol und Divinylbenzol, Methylmethacrylat und Äthylenglykoldimethacrylat oder 4-Vinylpyridin und Äthylenglykoldimethacrylat gebildet sind.

4. Topische Zusammensetzung nach einem der vorhergehenden Ansprüche, bei der die genannten festen Teilchen mit einem Vehikel kombiniert sind, um eine Zusammensetzung zu bilden, die aus flüssigen, halbfesten und festen Zusammensetzungen ausgewählt ist.

5. Topische Zusammensetzung nach einem der vorhergehenden Ansprüche, bei der das genannte Imprägniermittel von etwa 5% bis etwa 65% davon ausmacht.

6. Verfahren zur Herstellung einer topischen Zusammensetzung nach einem der Ansprüche 1 bis 5, umfassend:
   (a) das Bilden fester Teilchen, die ein im wesentlichen kontinuierliches Netzwerk aus Poren enthält, die zur Außenseite der genannten Teilchen offen sind; und
   (b) das Imprägnieren der genannten Poren der genannten festen Teilchen mit einem Imprägniermittel.

7. Verfahren nach Anspruch 6, worin Schritt (a) umfaßt:
   (i) das Kombinieren einer Monomerzusammensetzung mit einem Porogen, das eine im wesentlichen mit Wasser unmischbare Flüssigkeitspezies ist, die bezogen auf die genannte Monomerzusammensetzung inert ist, um eine homogene, im wesentlichen mit Wasser unmischbare flüssige Lösung zu bilden;
   (ii) das Suspendieren der genannten flüssigen Lösung in einer wässerigen Lösung, um eine Dispersion zu bilden;
   (iii) das Polymerisieren der genannten Monomerzusammensetzung in der genannten Dispersion, um feste Perlen zu bilden, welche die genannte mit Wasser unmischbare flüssige Chemikalienspezies enthalten;
   (iv) das Gewinnen der genannten festen Perlen aus der genannten Dispersion; und (v) das Extrahieren der genannten mit Wasser unmischbaren flüssigen Chemikalienspezies aus den genannten festen Perlen.

8. Verfahren zur Herstellung einer topischen Zusammensetzung, die feste Teilchen umfaßt, die ein im wesentlichen kontinuierliches, unzusammendrückbares Netzwerk aus Poren, die zur Außenseite der genannten Teilchen offen sind, und ein innerhalb der genannten Poren gehaltenes Imprägniermittel enthält; wobei das genannte Verfahren umfaßt:

(a) das Bilden der genannten Teilchen durch Suspensionspolymerisation in Gegenwart eines Porogens, sodaß poröse Teilchen mit dem Porogen in den Poren gebildet werden, wobei die genannten Teilchen im wesentlichen Kugelgestalt aufweisen, aus einem vernetzten Polymer gebildet sind und einen durchschnittlichen Durchmesser von etwa 10 µm bis etwa 40 µm, ein Gesamtporenvolumen von etwa 0,1 ml/g bis etwa 2,0 ml/g, eine Oberfläche von etwa 20 $m^2$/g bis etwa 200 $m^2$/g und einen durchschnittlichen Porendurchmesser von etwa 0,003 bis etwa 1,0 µm aufweisen;
(b) das Extrahieren des Porogens aus den Poren; und
(c) das Imprägnieren der Poren mit dem genannten Imprägniermittel, und worin das genannte Imprägniermittel wahlweise in einem Lösungsmittel vorliegt und eines oder mehrere aus Benzoylperoxid, Retinol, 3-Dihydroretinol, Retinsäuren, Retinaldehyden, Retinsäureestern und aromatischen Derivaten davon, Minoxidil, Squalan und Squalen umfaßt.

**9.** Verfahren zur kosmetischen Behandlung einer Epidermisregion, wobei das genannte Verfahren das Auftragen einer topischen Zusammensetzung nach einem der Ansprüche 1 bis 5 auf die genannte Epidermisregion umfaßt, wobei das genannte Imprägniermittel ein kosmetisches Behandlungsmittel umfaßt.

**Revendications**

**1.** Composition topique comprenant des particules solides contenant un réseau sensiblement continu et ne pouvant s'affaisser de pores ouverts vers l'extérieur desdites particules et un imprégnant retenu à l'intérieur desdits pores,où ledit imprégnant est facultativement dans un solvant et comprend un ou plusieurs parmi : peroxyde de benzoyle ; rétinol 3-dihydrorétinol acides rétinoïques, aldéhydes rétinoïques, esters d'acide rétinoïque et leurs dérivés aromatiques ; minoxidil ; squalane ; et squalène ; préparée par :
(a) formation desdites particules par polymérisation en suspension en présence d'un porogène de manière à former des particules poreuses avec le porogène dans les pores ;
(b) extraction du porogène des pores ; et
(c) imprégnation des pores par ledit imprégnant et où lesdites particules solides sont sensiblement de forme sphérique, sont formées d'un polymère réticulé ayant un diamètre moyen d'environ 10 microns à environ 40 µm, ayant un volume total des pores d'environ 0,1 ml/g à environ 2,0 ml/g, ayant une aire superficielle d'environ 20 $m^2$/g à environ 200 $m^2$/g et ayant un diamètre moyen des pores d'environ 0,003 à environ 1,0 µm.

**2.** Composition topique selon la revendication 1, où ledit imprégnant est dans un solvant choisi parmi des éthers, alcools, aromatiques et alcanes.

**3.** Composition topique selon la revendication 1 ou 2, où lesdites particules solides sont formées d'un copolymère de styrène et de divinylbenzène ; ou de méthacrylate de méthyle et de diméthacrylate d'éthylène glycol ou de 4-vinylpyridine et de diméthacrylate d'éthylène glycol.

**4.** Composition topique selon toute revendication précédente, où lesdites particules solides sont combinées avec un véhicule pour former une composition choisie parmi des compositions fluides, semi-solides et solides.

**5.** Composition topique selon l'une quelconque des revendications précédentes, où ledit imprégnant en forme environ 5% à environ 65%.

**6.** Méthode de préparation d'une composition topique selon l'une quelconque des revendications 1 à 5 comprenant:
(a) la formation de particules solides contenant un réseau sensiblement continu de pores ouverts vers l'extérieur desdites particules ; et
(b) l'imprégnation desdits pores desdites particules solides d'un imprégnant.

**7.** Méthode selon la revendication 6, où l'étape (a) comprend :
(i) la combinaison d'une composition monomère avec un porogène qui est sensiblement d'une espèce liquide immiscible dans l'eau qui est inerte par rapport à ladite composition monomère, pour former une solution liquide homogène sensiblement immiscible dans l'eau ;

(ii) la mise en suspension de ladite solution liquide dans une solution aqueuse pour former une dispersion ;
(iii) la polymérisation de ladite composition monomère dans ladite dispersion pour former des perles solides contenant ladite espèce chimique liquide immiscible dans l'eau ;
(iv) la récupération desdites perles solides de ladite dispersion ; et (v) l'extraction de ladite espèce chimique liquide immiscible dans l'eau desdites perles solides.

**8.** Méthode de préparation d'une composition topique comprenant des particules solides contenant un réseau sensiblement continu et ne pouvant s'affaisser de pores ouverts vers l'extérieur desdites particules et un imprégnant retenu à l'intérieur desdits pores, ladite méthode comprenant :
(a) la formation desdites particules par polymérisation en suspension en présence d'un porogène de manière que les particules poreuses avec le porogène dans les pores soient formées, lesdites particules étant sensiblement de forme sphérique, formées d'un polymère réticulé et ayant un diamètre moyen d'environ 10 $\mu$m à environ 40 $\mu$m, un volume total des pores d'environ 0,1 ml/g à environ 2,0 ml/g une aire superficielle d'environ 20 $m^2/g$ à environ 200 $m^2/g$ et un diamètre moyen des pores d'environ 0,003 à environ 1,0 $\mu$m ;
(b) l'extraction du porogène des pores ; et
(c) l'imprégnation des pores par ledit imprégnant et où ledit imprégnant est facultativement dans un solvant et comprend un ou plusieurs parmi le peroxyde de benzoyle; le rétinol, le 3-dihydrorétinol, les acides rétinoïques, les aldéhydes rétinoïques, les esters d'acide rétinoïque et leurs dérivés aromatiques ; le minoxidil ; le squalane ; et le squalène.

**9.** Méthode pour le traitement cosmétique d'une région de l'épiderme, ladite méthode comprenant l'application, à ladite région de l'épiderme, d'une composition topique selon l'une quelconque des revendications 1 à 5, ledit imprégnant comprenant un agent de traitement cosmétique.